Europäisches Patentamt

European Patent Office

Office européen des brevets

(19)

(11) Numéro de publication: **0 315 545**
**A1**

(12) **DEMANDE DE BREVET EUROPEEN**

(21) Numéro de dépôt: **88402777.2**

(22) Date de dépôt: **04.11.88**

(51) Int. Cl.⁴: **G 06 F 15/20**

(30) Priorité: **05.11.87 FR 8715368**

(43) Date de publication de la demande:
**10.05.89 Bulletin 89/19**

(84) Etats contractants désignés:
**DE FR GB IT NL SE**

(71) Demandeur: **CENTRE NATIONAL DE LA RECHERCHE SCIENTIFIQUE**
**15, quai Anatole France**
**F-75007 Paris (FR)**

(72) Inventeur: **Lumeau, Jean Bernard**
**1, Allée du Lot**
**F-31770 Colomiers (FR)**

(74) Mandataire: **Joly, Jean-Jacques et al**
**CABINET BEAU DE LOMENIE 55, rue d'Amsterdam**
**F-75008 Paris (FR)**

(54) **Dispositif de stéréolocalisation spatiale de sources d'activités cérébrales.**

(57) Des signaux électroencéphalographiques (EEG) recueillis par un ensemble d'électrodes (10) disposées en des emplacements prédéterminés du scalp sont convertis sous forme numérique, et transmis à une unité de traitement comprenant : des moyens d'estimation optimale d'une matrice interspectrale des signaux EEG recueillis ; des moyens de mémorisation dans lesquels sont enregistrées des informations relatives à un modèle de propagation et permettant de caractériser au moins un vecteur source définissant des relations, fonctions de paramètres de position de la ou chaque source, dans le domaine fréquentiel, entre un signal émis par une source dipolaire ou chaque source et les signaux disponibles sur le scalp, le modèle de propagation étant un modèle à trois discontinuités comprenant le cerveau, le crâne et le scalp ; et des moyens de traitement de la matrice interspectrale estimée débarrassée du bruit pour, d'une part, identifier les sources d'activités cérébrales contribuant aux signaux EEG recueillis et déterminer leur nombre et, d'autre part, estimer les coordonnées de chaque source identifiée.

EP 0 315 545 A1

**Description**

### Dispositif de stéréolocalisation spatiale de sources d'activités cérébrales

La présente invention a pour objet un dispositif de stéréolocalisation spatiale de sources d'activités cérébrales, c'est-à-dire un dispositif permettant de localiser dans le volume cérébral, par rapport à un système de coordonnées spatiales, une ou plusieurs sources à l'origine d'activités électriques cérébrales spécifiques.

Un domaine d'application particulier de l'invention est la localisation spatiale d'une activité cérébrale pathologique, par exemple un foyer épileptique.

Il est connu de réaliser une cartographie cérébrale à partir de signaux électroencéphalographiques (signaux EEG) recueillis au moyen d'électrodes implantées en différents endroits du scalp d'un patient. La cartographie cérébrale consiste à établir des cartes illustrant la répartition spatiale de la puissance d'émission à différentes fréquences par des nuances de gris ou des couleurs différentes.

Un dispositif d'analyse spectrale permettant d'obtenir de bons résultats en cartographie cérébrale a déjà été décrit dans la demande de brevet français No 87 07 600. Dans cette demande, il est notamment décrit un dispositif d'estimation d'une matrice interspectrale de signaux EEG recueillis par des électrodes, par mise en oeuvre, sur les signaux EEG, d'un algorithme récursif de blanchiment et décorrélation spatiale avec optimisation du compromis biais-variance pour chacun des éléments de la matrice.

La cartographie cérébrale permet de fournir des informations précieuses quant à l'existence de sources d'activités cérébrales et à leur emplacement approximatif. Toutefois, en particulier lorsqu'une exérèse doit être pratiquée, il est nécessaire de localiser avec précision la source d'activité cérébrale pathologique. A l'heure actuelle, cette localisation est réalisée dans la majorité des cas par implantation d'électrodes au sein de la masse cérébrale, à travers la boîte cranienne, ce qui est particulièrement traumatisant.

Aussi, la présente invention a-t-elle pour but de fournir un dispositif permettant, à partir d'une matrice interspectrale de signaux EEG recueillis, non seulement l'identification de sources d'activités cérébrales, mais aussi la localisation spatiale précise de ces sources au sein du volume cérébral.

Ainsi, l'invention vise à fournir un dispositif grâce auquel, en particulier, la localisation de foyers épileptiques peut être réalisée de façon non traumatisante, ce dispositif n'étant pas limité à cette application mais devant permettre, plus généralement, la localisation de toute source d'activité cérébrale pathologique ou non.

Conformément à l'invention, le dispositif de stéréolocalisation comporte :

(a) un ensemble d'électrodes destinées à être disposées en des emplacements prédéterminés du scalp pour recueillir des signaux EEG,

(b) des moyens convertisseurs pour convertir les signaux EEG recueillis sous forme numérique, et

(c) une unité de traitement comprenant :

- des moyens d'estimation d'une matrice interspectrale des signaux EEG recueillis,

- des moyens de mémorisation dans lesquels sont enregistrées des informations relatives à un modèle de propagation et permettant de caractériser au moins un vecteur source définissant dans le domaine fréquentiel des relations, fonctions de paramètres de position de la ou chaque source, entre un signal émis par une source dipolaire ou chaque source et les signaux disponibles sur le scalp, le modèle de propagation étant un modèle à trois discontinuités comprenant le cerveau, le crâne et le scalp, et

- des moyens de traitement de la matrice interspectrale estimée pour, d'une part, identifier les sources d'activités cérébrales contribuant aux signaux EEG recueillis et déterminer leur nombre et, d'autre part, estimer les coordonnées de chaque source identifiée.

De préférence, les moyens de traitement de la matrice interspectrale comprennent des moyens d'estimation des vecteurs propres correspondant aux valeurs propres de la matrice non liées aux sources identifiées, et des moyens de détermination des coordonnées de position de sources pour lesquelles le vecteur source correspondant est orthogonal auxdits vecteurs propres.

Le traitement de la matrice interspectrale pour l'identification et la localisation des sources est effectué sur une matrice débarrassée du bruit.

Une matrice interspectrale estimée non bruitée peut être obtenu par la technique du moyennage, dans les cas où l'activité cérébrale est provoquée par des stimulations, celles-ci étant répétées pour faire émerger le signal par rapport au bruit.

Dans d'autres cas (notamment pour des sources d'activités cérébrales pathologiques), la technique du moyennage n'est pas toujours utilisable. Comme indiqué plus loin de façon détaillée, on peut parvenir à estimer une matrice interspectrale débarrassée de son bruit à partir de la matrice de cohérence associée à la matrice interspectrale estimée.

L'invention sera mieux comprise à la lecture de la description faite ci-après, à titre indicatif mais non limitatif, en référence aux dessins annexés sur lesquels :

- la figure 1 illustre très schématiquement un mode de réalisation d'un dispositif de stéréolocalisation conforme à l'invention,

- la figure 2 est un organigramme montrant de façon générale les phases successives mises en oeuvre par le dispositif conforme à l'invention,

- la figure 3 illustre un modèle de propagation d'ondes dans un milieu composite avec trois discontinuités de conductivité, et

- les figures 4A à 4E illustrent la localisation d'une source par mise en oeuvre de la méthode du projecteur orthogonal sur les données fournies par la matrice interspectrale débarrassée du bruit, au moyen du dispositif conforme à l'invention.

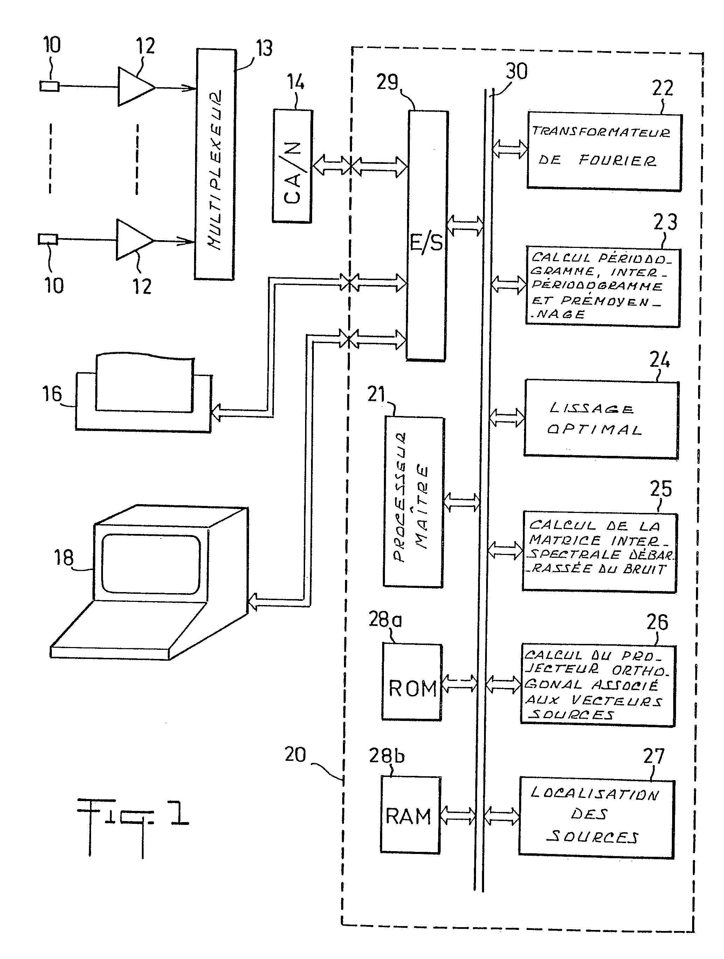

Dans le dispositif illustré par la figure 1, les signaux EEG sont recueillis au moyen d'électrodes de scalp 10, puis amplifiés au moyen d'amplificateurs 12, multiplexés par un multiplexeur 13 et convertis sous forme numérique au moyen d'un convertisseur analogique-numérique 14.

Avantageusement, les amplificateurs 12 sont du type de celui décrit dans la demande de brevet français No 2 564 657.

Les signaux numérisés sont traités au moyen d'une unité de traitement 20 qui, compte-tenu du volume de traitements à effectuer, est de préférence du type à architecture multiprocesseur organisé autour d'un bus commun 30. L'unité de traitement 20 comprend :
- un processeur maître 21 assurant le séquencement des opérations et le calcul final de la matrice interspectrale des signaux EEG recueillis,
- un transformateur de Fourier rapide 22,
- un processeur 23 effectuant des calculs de périodogrammes et interpériodogrammes, des prémoyennages et des transformations logarithmiques directes et inverses, intervenant dans le calcul de la matrice interspectrale,
- un processeur 24 effectuant des opérations de lissage optimal,
- un processeur 25 de calcul de la matrice interspectrale débarrassée du bruit,
- un processeur 26 de calcul de projecteur orthogonal associé à des vecteurs sources,
- un processeur de localisation 27 effectuant le calcul de coordonnées de sources d'activités cérébrales identifiées,
- des mémoires mortes (ROM) 28a destinées à la mémorisation des programmes et d'informations représentatives de vecteurs sources pour un modèle de propagation prédéterminé entre source et électrodes,
- des mémoires vives (RAM) 28b pour la mémorisation temporaire de données, et
- un circuit d'entrées/sorties 29.

Différents périphériques sont connectés à l'unité de traitement 20 dont, notamment, une imprimante 16 et une console de visualisation 18.

Les phases principales du programme de localisation de sources d'activités cérébrales mis en oeuvre par le dispositif décrit ci-avant sont indiquées sur la figure 2.

Estimation de la matrice interspectrale

La phase 101 d'estimation de la matrice interspectrale des signaux EEG recueillis est mise en oeuvre de la façon décrite dans la demande de brevet français No 87 07 600 déjà citée.

Essentiellement, l'estimation de la matrice interspectrale est réalisée par mise en oeuvre d'un algorithme itératif de blanchiment et décorrélation sur les transformées de Fourier des signaux EEG recueillis. Le blanchiment d'un signal est effectué après estimation de son autospectre par la méthode de lissage du Log-périodogramme après détermination d'une fenêtre de pondération optimale. Le passage au Log-périodogramme est de préférence précédé par un prémoyennage du périodogramme. La décorrélation de deux signaux est effectuée après estimation de leur cohérence par la méthode de lissage de l'inter-périodogramme des deux signaux blanchis après détermination d'une fenêtre de pondération optimale.

De par ses propriétés, et pour un modèle de propagation donné entre sources d'activités cérébrales et électrodes disposées sur le scalp, la matrice interspectrale estimée et débarrassée de son bruit permet de déterminer le nombre de sources décorrélées et leur localisation dans le volume cérébral par rapport à un système de coordonnées.

Les signaux EEG recueillis ont un très faible rapport signal/bruit. Une utilisation de la matrice interspectrale estimée pour identifier et localiser des sources de façon sûre n'est possible que dans la mesure où la matrice estimée est non bruitée.

L'élimination du bruit peut résulter de la mise en oeuvre de la technique du moyennage, c'est-à-dire en effectuant la moyenne de résultats obtenus par suite de stimulations répétées des sources.

Lorsque la technique du moyennage n'est pas applicable, par exemple lorsque l'activité des sources n'est pas liée à des stimulations extérieures contrôlées et n'a pas le caractère répétitif nécessaire pour faire émerger le signal par rapport au bruit par moyennage, il est nécessaire de débarrasser la matrice interspectrale estimée du bruit (phase 102).

Estimation de la matrice interspectrale débarrassée du bruit

Dans l'hypothèse de bruits décorrélés de puissance quelconque sur les électrodes, hypothèse vérifiée dans la majorité des applications neurophysiologiques, la matrice interspectrale $\overset{\circ}{\Gamma}_x$ peut se mettre sous la forme de la somme d'une matrice signal $\overset{\circ}{\Gamma}_y$ de rang $r_o$ inférieur à K et d'une matrice de bruit $\overset{\circ}{\Gamma}_\epsilon$ diagonale mais non scalaire : $\overset{\circ}{\Gamma}_x = \overset{\circ}{\Gamma}_y + \overset{\circ}{\Gamma}_\epsilon$,

$r_o$ étant la dimension du sous-espace sources (nombres de sources décorrélées) et K le nombre d'électrodes, tandis que
- la notation ° signifie que l'on se trouve dans le domaine fréquentiel,
- la notation x se rapporte aux signaux EEG recueillis,

3

- la notation y se rapporte à la composante des signaux EEG provenant de la ou des sources à identifier et localiser, et
- la notation $\varepsilon$ se rapporte à la composante de bruit des signaux EEG.

L'estimation de la matrice interspectrale débarassée du bruit consiste à rechercher une estimée $\hat{\hat{\Gamma}}_y$ de $\hat{\Gamma}_y$ à partir de l'estimée $\hat{\hat{\Gamma}}_x$ obtenue précédemment dans la phase 101 (la notation $\wedge$ signifie qu'il s'agit d'une estimée).

On peut montrer qu'il suffit, pour déterminer une matrice d'ordre K de façon unique à partir de ses éléments non diagonaux, que le rang $r_o$ de cette matrice soit inférieur ou égal à (K/2 - 1) et que tout mineur $(r_o, r_o)$ soit de rang $r_o$.

Mais, en pratique, en raison du bruit et du biais introduit par l'estimation de la matrice interspectrale, on ne peut affirmer qu'il existe une matrice de rang $r_o$ dont les éléments non diagonaux sont identiques à ceux de $\hat{\hat{\Gamma}}_x$. De la sorte, et bien qu'en principe seuls les éléments diagonaux de $\hat{\Gamma}_x$ sont affectés par le bruit, on ne peut rechercher qu'une estimée de $\hat{\Gamma}_y$ à partir des éléments non diagonaux de $\hat{\hat{\Gamma}}_x$.

Une approche par minimisation d'un critère quadratique portant sur la différence entre les éléments non diagonaux de $\hat{\hat{\Gamma}}_x$ et $\hat{\hat{\Gamma}}_y$ montre que l'on peut se ramener à un traitement de la matrice de cohérence $\hat{\hat{C}}_x$ associée à $\hat{\hat{\Gamma}}_x$, c'est-à-dire à la matrice dont les éléments

$$\hat{\hat{C}}_{x_{ij}}$$

sont donnés par :

$$\hat{\hat{C}}_{x_{ij}} = \hat{\hat{\gamma}}_{x_{ij}} \left( \hat{\gamma}_{x_{ii}} \, \hat{\gamma}_{x_{jj}} \right)^{-1/2}$$

les

$$\hat{\hat{\gamma}}_{x_{ij}}$$

étant les éléments de $\hat{\hat{\Gamma}}_x$, i étant l'indice de ligne et j l'indice de colonne.

En effet, un critère quadratique de la forme

$$Q = \sum_{i \neq j} \left( \hat{\hat{\gamma}}_{y_{ij}} - \hat{\hat{\gamma}}_{x_{ij}} \right)^2 \hat{w}(i,j),$$

dans lequel $\hat{w}(i, j)$ est la pondération, c'est-à-dire :

$$\hat{w}(i,j) = \left( \hat{\gamma}_{x_{ii}} \, \hat{\gamma}_{x_{jj}} \right)^{-1}$$

peut se ramener, en remplaçant la pondération par la valeur approchée

$$\left(\hat{\overset{\circ}{\gamma}}_{x_{ii}}\hat{\overset{\circ}{\gamma}}_{x_{jj}}\right)^{-1}, \text{à :}$$

$$Q = \sum_{i\neq j}\left[\frac{\overset{\circ}{\hat{\gamma}}_{y_{ij}}-\overset{\circ}{\hat{\gamma}}_{x_{ij}}}{\sqrt{\overset{\circ}{\hat{\gamma}}_{x_{ii}}\,\overset{\circ}{\hat{\gamma}}_{x_{jj}}}}\right]^2, \text{ou} \quad Q^{op} = Q \left/ \sum_{i\neq j}\left[\frac{\overset{\circ}{\hat{\gamma}}_{x_{ij}}}{\sqrt{\overset{\circ}{\hat{\gamma}}_{x_{ii}}\,\overset{\circ}{\hat{\gamma}}_{x_{jj}}}}\right]^2\right.,$$

$Q^{op}$ représentant la grandeur $Q$ normalisée de manière à faire apparaître une erreur relative, d'où introduction de notion de cohérence.

Le traitement mis en oeuvre sur la matrice de cohérence estimée $\hat{\overset{\circ}{C}}_x$ revient donc à rechercher, dans un ensemble $\{\overset{\circ}{C}(r)\}$ de matrices définies non négatives de rang $r$ inférieur ou égal à $r_0$, la matrice $C_m(r)$ qui minimise le critère

$$Q^{op}(r) = \frac{\sum_{i\neq j}|\overset{\circ}{c}_{ij}(r) - \hat{\overset{\circ}{c}}_{x_{ij}}|^2}{\sum_{i\neq j}|\hat{\overset{\circ}{c}}_{x_{ij}}|^2}.$$

Cette recherche est effectuée à l'aide d'un algorithme de type gradient matriciel à pas de convergence optimal.

D'une façon générale, le processus consiste à rechercher, par valeur croissante de $r$, dans la suite des $\{Q_m^{op}(r)\}$, c'est- à-dire les minima de $Q^{op}(r)$, le rang $r_0$ correspondant à la plus faible valeur de $r$ pour laquelle $Q^{op}(r)$ est voisin d'une borne $Q_M^{op}(r)$ définie par :

$$Q_M^{op}(r) = \frac{\sum_{i\neq j}\dfrac{1}{L_c}}{\sum_{i\neq j}|\hat{\overset{\circ}{c}}_{x_{ij}}|^2}\quad,$$

$L_e$ étant la largeur équivalente de la fenêtre de lissage fournie par la méthode optimale d'estimation spectrale des éléments

$$\overset{\circ}{c}_{x_{ij}}$$

de la matrice $\overset{\circ}{C}_x$, comme décrit dans la demande de brevet français No 87 07 600 déjà citée. Pour un faible rapport signal sur bruit, la borne $Q_M^{op}(r)$ tend vers une valeur $Q_0^{op}(r)$ définie par :
$Q_0^{op}(r) = [(k-1-r)/(k-1)]\, Q_m^{op}(r)$.

Si $\overset{\circ}{C}(r_0)$ est la matrice qui correspond au minimum $Q_m^{op}(r_0)$, la matrice $\hat{\Gamma}_y$ débarrassée du bruit sera estimée par la matrice $\hat{\overset{\approx}{\Gamma}}_y$ d'éléments :

$$\hat{\overset{\circ}{\gamma}}_{x_{ij}} = \sqrt{\hat{\overset{\circ}{\gamma}}_{x_{ii}}\,\hat{\overset{\circ}{\gamma}}_{y_{jj}}\,\overset{\circ}{c}_{ij}(r_0)}\quad.$$

L'algorithme de type gradient matriciel pour la recherche du minimum sera maintenant décrit.

La différentielle $dQ^{op}$ (r) du critère $Q^{op}$ (r), qui est nulle au minimum de ce critère, s'exprime par

$$dQ^{op}(r) = \left[ \sum_{i \neq j} (\overset{o}{c}_{ij}(r) - \overset{\wedge o}{c}_{x_{ij}})^2 \, d\overset{o*}{c}_{ij}(r) + (\overset{o*}{c}_{ji}(r) - \overset{\wedge o *}{c}_{x_{ji}}) \, d\overset{o}{c}_{ji}(r) \right] \Big/ \left[ \sum_{i \neq j} |\overset{\wedge o}{c}_{x_{ij}}|^2 \right]$$

ce qui, compte-tenu du caractère hermitien des matrices $\overset{o}{C}$ (r) et $\overset{\wedge}{\overset{o}{c}}_x$, donne :

$$dQ^{op}(r) = \frac{2 \sum\limits_{i \neq j} (\overset{o}{c}_{ij}(r) - \overset{\wedge o}{c}_{x_{ij}}) \, d\overset{o*}{c}_{ij}(r)}{\sum\limits_{i \neq j} |\overset{\wedge o}{c}_{x_{ij}}|^2} \quad .$$

Si l'on désigne par $\overset{o}{H}$ (r) la matrice définie par les éléments

$$\overset{o}{h}_{ii}(r) = 0 \quad \text{et} \quad \overset{o}{h}_{ij}(r) = \overset{o}{c}_{ij}(r) - \overset{\wedge o}{c}_{x_{ij}}$$

pour i différent de j, et en raisonnant sur le numérateur Q (r) de $Q^{op}$ (r) pour alléger les notations, on obtient :
$dQ(r) = 2 \, tr \{ \overset{o}{H}(r) \, d\overset{o}{C}(r) \}$.
ce qui, la matrice $\overset{o}{C}$ (r) étant factorisée sous la forme $\overset{o}{C}(r) = \overset{o}{M}(r) \, \overset{o}{M}^+(r)$ , conduit à la relation :
$dQ(r) = 2tr \{ \overset{o}{H}(r) \, \overset{o}{M}(r) \, d\overset{o}{M}(r)^+ \} + 2tr \{ \overset{o}{H}(r) \, d\overset{o}{M}(r) \, \overset{o}{M}(r)^+ \}$
la notation "tr" signifiant "trace de".

La différentielle dQ(r), et, par suite la différentielle $dQ^{op}$ (r) sont nulles si $\overset{o}{H}(r)\overset{o}{M}(r) = 0$. Pour trouver le minimum ainsi défini, on forme un algorithme de type gradient tel que
$\delta \overset{o}{M}(r) = - \mu \, \overset{o}{H}(r) \, \overset{o}{M}(r)$ avec $0 < \mu \ll 1$.

Pour $\mu$ suffisamment petit, l'accroissement $\delta Q(r)$ de Q(r) peut être assimilé à sa différentielle, ce qui donne :
$\delta Q(r) = - 4 \, \mu tr \{ \overset{o}{H} \, \overset{o}{M}(r) \, \overset{o}{M}(r)^+ \, \overset{o}{H}^+ \}$ .

La trace de $\{\overset{o}{H}\overset{o}{M}(r) \, \overset{o}{M}(r)^+\overset{o}{H}^+\}$ représente la somme des carrés des modules des éléments de la matrice $\overset{o}{H}\overset{o}{M}(r)$. Elle est donc positive ou nulle et ne peut être nulle que si tous les éléments de la matrice $\overset{o}{H}\overset{o}{M}(r)$ sont nuls, c'est-à-dire pour un extrêmum de Q(r) ou, de façon équivalente, de $Q^{op}(r)$.

Pour une suite de matrices $\{M_R(r)\}$, l'algorithme peut donc être défini par :
$\overset{o}{M}_{k+1}(r) = \overset{o}{M}_k(r) - \mu_k \, \overset{o}{H}_k \, \overset{o}{M}_k(r)$ .

Si l'on considère la suite correspondante des valeurs $Q_k(r)$ du critère, pour $\mu_k$ suffisamment petit, l'on obtient :
$Q_{k+1}(r) - Q_k(r) = - 4 \, \mu_k \, tr\{ \overset{o}{H}_k(r)\overset{o}{M}_k(r) \, \overset{o}{M}_k(r)^+ \, \overset{o}{H}_k(r)^+\}$.

Cette variation est négative tant que la matrice $\overset{o}{H}_k(r)\overset{o}{M}_k(r)$ ne se réduit pas à la matrice nulle. Les valeurs $\{Q_k (r)\}$ du critère forment donc und suite décroissante qui converge vers un minimum local de Q(r) ou $Q^{op}(r)$.

La vitesse de convergence vers le minimum peut être accélérée en recherchant le pas de convergence optimal, c'est-à-dire, dans la direction du gradient, le $\mu_k$ qui donne le minimum du critère. Le $\mu_k$ est déterminé de façon approchée en faisant l'hypothèse d'une variation localement quadratique. A partir de la relation ci-dessus donnant $\overset{o}{M}_{k+1}(r)$, on peut écrire :

$$\overset{o}{M}_{k+1} \overset{o}{M}_{k+1}^+ = \overset{o}{M}_k \overset{o}{M}_k^+ - \mu_k(\overset{o}{H}_k \overset{o}{M}_k \overset{o}{M}_k^+ + \overset{o}{M}_k \overset{o}{M}_k^+ \overset{o}{H}_k^+) + \mu_k^2 \overset{o}{H}_k \overset{o}{M}_k \overset{o}{M}_k^+ \overset{o}{H}_k^+.$$

En négligeant le terme en $\mu_k^2$ relatif au carré de l'erreur, on a :

$$\overset{\circ}{M}_{k+1} \overset{\circ}{M}_{k+1}^{+} - \overset{\wedge}{\overset{\circ}{C}} \cong \overset{\circ}{M}_{k} \overset{\circ}{M}_{k}^{+} - \overset{\wedge}{\overset{\circ}{C}} - \mu_{k} \overset{\circ}{D}_{k}$$

avec $\quad \overset{\circ}{D}_{k} = \overset{\circ}{H}_{k} \overset{\circ}{M}_{k} \overset{\circ}{M}_{k}^{+} + \overset{\circ}{M}_{k} \overset{\circ}{M}_{k}^{+} \overset{\circ}{H}_{k}^{+}.$ Par suite :

$$\begin{cases} \overset{\circ}{H}_{k+1}(\mu_{k}) \cong \overset{\circ}{H}_{k} - \mu_{k} \overset{\circ}{D}_{k} \quad \text{pour } i \neq j, \\[2mm] \overset{\circ}{H}_{k+1}^{2}(\mu_{k}) \cong \overset{\circ}{H}_{k}^{2} - \mu_{k} \overset{\circ}{H}_{k} \overset{\circ}{D}_{k} - \mu_{k} \overset{\circ}{D}_{k} \overset{\circ}{H}_{k} + \mu_{k}^{2} \overset{\circ}{D}_{k}^{2}, \\[2mm] \text{tr}\{[\overset{\circ}{H}_{k+1}^{2}(\mu_{k})]'\} \cong 2\text{tr} \{[\overset{\circ}{H}_{k} - \mu_{k} \overset{\circ}{D}_{k}] \cdot \overset{\circ}{D}_{k}\}, \end{cases}$$

De la dernière relation, en annulant la dérivée, il vient :

$$\mu_{k} = \frac{\text{tr}\{\overset{\circ}{H}_{k} \overset{\circ}{D}_{k}\}}{\text{tr}\{\overset{\circ}{D}_{k}^{2}\}} \quad .$$

ce qui permet de calculer le $\mu_k$ optimal à chaque itération de k.

On décrira maintenant un processus permettant d'initialiser l'algorithme afin d'éviter une éventuelle convergence vers une valeur erronnée par suite d'un mauvais choix de conditions initiales (c'est-à-dire les $\overset{\circ}{c}_{ij}(r)$. Ce processus consiste en une méthode simplifiée servant à estimer, par valeur inférieure, l'ordre de grandeur du rang recherché et à fournir une estimée de $\overset{\circ}{M}(r)$ pour initialiser l'algorithme précédent.

La matrice $\overset{\wedge}{\overline{\overline{\varepsilon}}}$ étant définie non négative peut se décomposer de façon unique sous le forme d'un produit de matrices triangulaires avec éléments positifs sur la diagonale. Dans le cas d'une matrice de rang r inférieur à K, cette décomposition prend la forme suivante :

$$\overset{\wedge}{\overset{\circ}{C}} = \begin{bmatrix} \overset{\circ}{P}_{1} = B_{1} B_{1}^{+} & \overset{\circ}{P}_{2} = \overset{\circ}{B}_{1} \overset{\circ}{B}_{2}^{+} \\ \overset{\circ}{P}_{3} = \overset{\circ}{B}_{2} \overset{\circ}{B}_{1}^{+} & \overset{\circ}{P}_{4} = \overset{\circ}{B}_{2} \overset{\circ}{B}_{2}^{+} \end{bmatrix} = \begin{bmatrix} \overset{\circ}{B}_{1} & 0 \\ \overset{\circ}{B}_{2} & \end{bmatrix} \begin{bmatrix} \overset{\circ}{B}_{1}^{+} & \overset{\circ}{B}_{2}^{+} \\ 0 & \end{bmatrix}$$

Les éléments non diagonaux de $\overset{\wedge}{\overline{\overline{\varepsilon}}}$ étant connus (identique à ceux de $\overset{\wedge}{\hat{\varepsilon}}_{x}$), il suffit de déterminer les r premiers éléments diagonaux pour que la matrice soit complètement définie.

En effet, le bloc diagonal $\overset{\circ}{P}_1$ étant alors supposé connu, on peut, par un algorithme classique, le décomposer en un produit tel que $\overset{\circ}{P}_1 = \overset{\circ}{B}_1\overset{\circ}{B}^+_1$. Ainsi, le blanc $\overset{\circ}{B}_2$ est déterminé par la relation $\overset{\circ}{B}_2 = \overset{\circ}{P}_3[\overset{\circ}{B}^+_1]^{-1}$, ce qui permet de calculer les éléments diagonaux manquants de $\overset{\hat{\approx}}{c}$ à partir du produit matriciel :

$$\overset{\circ}{P}_4 = \overset{\circ}{B}_2\overset{\circ}{B} \quad .$$

La détermination du bloc diagonal et du rang de $\overset{\hat{\approx}}{c}$ débarrassée du bruit, donc la détermination des r premiers éléments diagonaux est réalisée comme suit.

On désigne par $\{\overset{\hat{\approx}}{v}(j)\}$ les vecteurs colonnes de $\overset{\hat{\approx}}{c}_x$ et par $\{\overset{\circ}{w}(j)\}$ les vecteurs formés par les $(K - r - 1)$ dernières composantes des $\{\overset{\approx}{v}(j)\}$, et par $\overset{\circ}{B}$ la matrice formée par r vecteurs :

$$\{\overset{\wedge}{\overset{\circ}{w}}(1), \overset{\wedge}{\overset{\circ}{w}}(2), ...., \overset{\wedge}{\overset{\circ}{w}}(r)\}$$

En général, le vecteur $\overset{\hat{\approx}}{w}(r + 1)$ ne s'exprimera pas comme combinaison linéaire de ces r vecteurs du fait que la matrice $\overset{\hat{\approx}}{c}_x$ est biaisée et bruitée. On définit alors

$$\overset{\sim}{\overset{\circ}{w}}(r+1) = \sum_{j=1}^{r} \overset{\sim}{\varsigma}(j) \; \overset{\wedge}{\overset{\circ}{w}}(j) \; ,$$

où les $\overset{\sim}{\varsigma}(j)$ sont déterminés en minimisant le critère

$$Q^{sp}(r) = \frac{|\overset{\wedge}{\overset{\circ}{w}}(r+1) - \overset{\sim}{\overset{\circ}{w}}(r+1)|^2}{|\overset{\wedge}{\overset{\circ}{w}}(r+1)|^2} \quad .$$

Si la matrice $\overset{\circ}{B}$ est de rang r, le vecteur paramètre correspondant au minimum est donné par $\overset{\sim}{\varsigma}(r) = [\overset{\circ}{B}^+ \overset{\circ}{B}]^{-1} \overset{\circ}{B}^+ \overset{\wedge}{\overset{\circ}{w}}(r+1)$ .

En considérant la matrice $\overset{\circ}{B}$ de rang r, les paramètres $\{\overset{\sim}{\varsigma}(j)\}$ sont les estimées des coefficients du $(r + 1)^{ème}$ vecteur colonne de la matrice de cohérence "débarrassée" du bruit en fonction des r premiers. Pour les indices de ligne $\{h\}$ où h est inférieur ou égal à r, les éléments diagonaux $\overset{\sim}{c}_{hh}$ sont donc estimés à partir de la relation :

$$\overset{\wedge}{\overset{\circ}{c}}_{xh(r+1)} = \sum_{j \neq h}^{r} \overset{\sim}{\varsigma}(j) \; \overset{\wedge}{\overset{\circ}{c}}_{xhj} + \overset{\sim}{\varsigma}(h) \; \overset{\sim}{\overset{\circ}{c}}_{hh}$$

où l'on supposera que $\overset{\sim}{\varsigma}(h)$ est différent de zéro.

Pour une première estimation de l'ordre, on peut raisonner sur la valeur du critère $Q^{sp}(r)$. Pour une matrice non perturbée $Q^{sp}(r)$ s'annulerait pour r supérieur ou égal à $r_0$. En présence d'erreur statistique, et comme, plus haut, pour le critère $Q^{op}(r)$, peut déterminer, en l'absence de biais, un seuil supérieur $Q_M^{sp}(r)$ pour l'espérance du majorant du minimum du critère $Q^{sp}(r)$. Ce seuil supérieur est donné par :

$$Q_M^{sp}(r) = \frac{(K-r-1) \; (1+|\overset{\sim}{\varsigma}(r)|^2) \; /L_e}{|\overset{\wedge}{\overset{\circ}{w}}(r+1)|^2} \geq Q_m^{sp}(r) \quad .$$

A faible rapport signal sur bruit, cette borne tend vers

$$Q_0^{sp}(r) = \frac{K-2r-1}{K-r-1} \, Q_M^{sp}(r) \, .$$

Dans la mesure où $Q_0^{sp}(r)$ est toujours un majorant du seuil d'arrêt, le rang estimé à partir de ce critère (qui ne fait intervenir qu'une sous-matrice de $\hat{\hat{c}}_x$) correspond bien à une estimation par défaut.

Le bloc $\overset{\circ}{P}_1$ de la matrice $\hat{\hat{c}}_x$ étant déterminé comme indiqué au sous-paragraphe précédent et les matrices $\overset{\circ}{B}_1$ et $\overset{\circ}{B}_2$, dont l'ensemble définit la matrice $\overset{\circ}{M}(r)$, étant calculées, on remarque que les matrices $\hat{\hat{c}}_x$ et $\overset{\circ}{C}(r)$ ($\overset{\circ}{C}(r) = \overset{\circ}{M}(r)\overset{\circ}{M}+(r)$) ne diffèrent que par le bloc $\overset{\circ}{P}_4$ ($\overset{\circ}{P}_4 = \overset{\circ}{B}_2 \overset{\circ}{B}_2^{:}$).

La matrice $\overset{\circ}{C}(r)$ étant déterminée entièrement à partir des $(r + 1)$ premières colonnes de $\hat{\hat{c}}$, les lignes et les colonnes sont ordonnées de façon à grouper dans ces colonnes les éléments pour lesquels le coefficient de cohérence est le plus grand.

Pour ce classement il suffit, par exemple, de s'intéresser à la sous-matrice inférieure et de :
- rechercher dans cette sous-matrice le coefficient de cohérence maximum

$$\overset{\circ}{\hat{C}}_{x \, i,j}$$

et, par une permutation de l'ordre des indices de la matrice, de le positionner sur la dernière ligne de la première colonne,
- combiner la première colonne aux suivantes de façon à faire apparaître des zéros sur la dernière ligne (à l'exception de l'élément de la première colonne),
- rechercher dans la matrice ainsi obtenu, privée de sa dernière ligne et de sa première colonne, l'élément maximum et, par une nouvelle permutation de l'ordre des indices de la matrice, positionner cet élément sur l'avant dernière ligne de la deuxième colonne, et ainsi de suite.

Ce classement conduit à une matrice triangulaire. Les éléments diagonaux de cette matrice sont rangés, de la gauche vers la droite, par valeur décroissante. Le passage par la recherche de combinaison entre les éléments de la matrice, dans ce classement, se justifie par le fait qu'il est nécessaire de s'assurer que la grandeur des coefficients de cohérence ne résulte pas de la proportionnalité de deux colonnes (ou de deux lignes) de la matrice $\hat{\hat{c}}_x$.

Le processus d'estimation de la matrice interspectrale débarrassée du bruit décrit ci-avant suppose seulement que les bruits sont décorrélés. Si l'on se place de plus dans l'hypothèse bruit blanc, l'estimation du sous-espace source peut encore être améliorée.

Dans le cas où tous les éléments de la matrice sont utilisés, le critère à minimiser s'écrit alors :

$$Q'^{op}(r) = \frac{\mathrm{tr}\{C(r)-\hat{C}_x\}^2}{\mathrm{tr}\{\hat{C}_x\}^2} = \frac{\displaystyle\sum_{i=r+1}^{K} \lambda^2(i)}{\displaystyle\sum_{i=1}^{K} \lambda^2(i)} \, ,$$

où $C(r)$ est une matrice de cohérence définie non négative de rang inférieur ou égal à $r$ et $\hat{C}_x$ la matrice de cohérence estimée et bruitée déduite de la matrice de covariance estimée et bruitée par la relation :

$$\hat{C}_{x_{ij}} = \frac{\hat{\gamma}_{x_{ij}}}{\sqrt{\hat{\gamma}_{x_{ii}} \, \hat{\gamma}_{x_{jj}}}} \, .$$

Sous l'hypothèse bruit blanc, la matrice $C(r)$ peut s'écrire :
$C(r) = M(r) \, M^+(r) \, + \, \sigma^2(r) \, \hat{D}_x^{-1} \, ,$

où M(r) M⁺(r) représente la matrice de cohérence débarrassée du bruit au rang r, $\sigma^2(r)$ la puissance du bruit blanc au rang r et $\hat{D}_x$ la matrice diagonale définie par :

$$\hat{D}_x = \begin{bmatrix} \hat{\gamma}_{x11} & & & 0 \\ & \cdot & & \\ & & \cdot & \\ & & & \cdot \\ 0 & & & \hat{\gamma}_{xkk} \end{bmatrix}.$$

Dans ces conditions, il est possible de déterminer le $\sigma^2(r)$ qui minimise $Q'^{op}(r)$,

$$\hat{\sigma}^2(r) = \frac{\text{tr}\{\hat{C}_x(r) - M(r)\,M^+(r)\}\,\hat{D}_x^{-1}}{\text{tr}\{\hat{D}_x^{-2}\}}.$$

Ainsi, si l'on pose
$$\hat{C}''_x = \hat{C}_x - \hat{\sigma}^2(r)\,\hat{D}_x^{-1}$$
le rang $r_o$ optimal est défini en minimisant le critère :

$$Q''^{op}(r) = \frac{\displaystyle\sum_{i,j}|c_{ij}(r) - \hat{c}''_{x_{ij}}|^2}{\displaystyle\sum_{i,j}|\hat{c}''_{x_{ij}}|^2}$$

au lieu du critère $Q^{op}(r)$. Le reste du processus décrit précédemment reste inchangé et s'applique directement.

Le calcul itératif sur r et sur $\hat{\sigma}^2_{(r)}$ permet d'identifier le sous-espace source en déterminant $r_o$, d'estimer la puissance du bruit blanc et, de plus, fournit une estimation de la matrice de covariance débarrassée du bruit.

L'estimation de la matrice interspectrale débarrassée du bruit permet d'évaluer le nombre $r_o$ de sources décorrélées (cette approche pouvant s'étendre au cas de sources corrélées) et de fournir, par le calcul des valeurs propres associées, une information relative à la puissance d'émission de ces sources. Mais l'estimation de la matrice interspectrale débarrassée du bruit permet aussi la localisation des sources, dans la mesure où un modèle de propagation entre sources et électrodes a été défini.

Définition d'un modèle de propagation

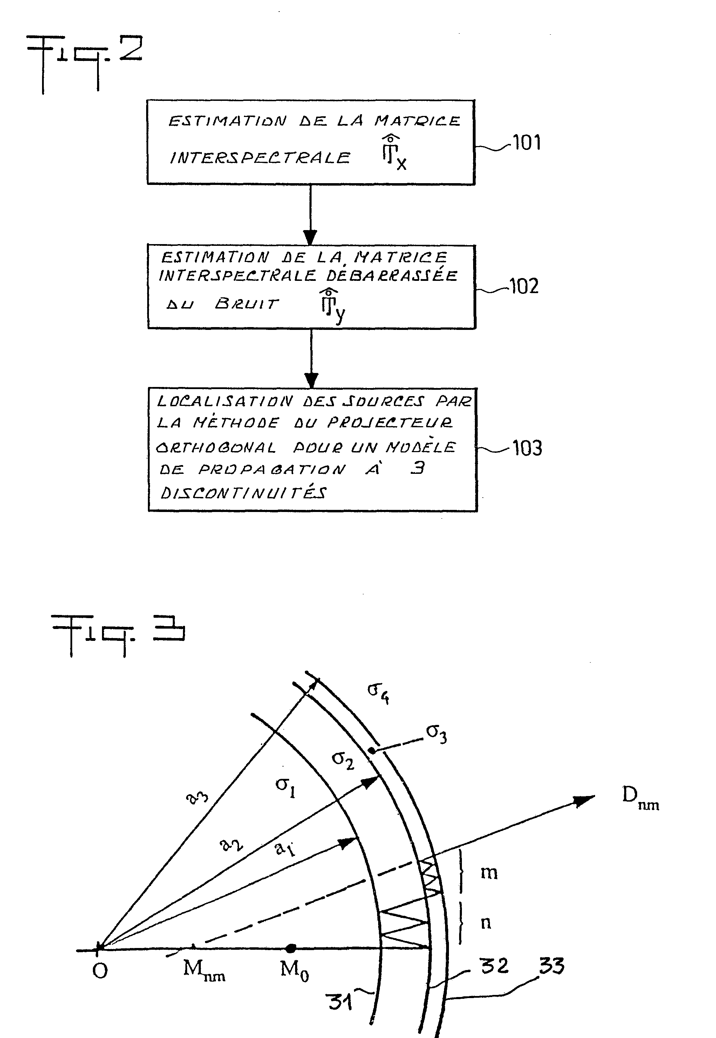

Le modèle utilisé ici est un modèle à trois discontinuités de conductivité électrique schématisé par trois sphères concentriques de rayons respectifs $a_1$, $a_2$, $a_3$ représentant le cerveau avec le liquide céphalo-rachidien, le crâne et le scalp (figure 3).

On se place dans l'hypothèse d'une charge ponctuelle placée en un point $M_o$ à distance b du centre O des sphères. Les discontinuités de conductivité sont très importante, puisque l'on peut atteindre un rapport de 100 entre la conductivité $\sigma_2$ du crâne et les conductivités $\sigma_1$ et $\sigma_3$ du cerveau et du scalp qui sont voisines l'une de l'autre, et puisque la conductivité $\sigma_4$ à l'extérieur du scalp est très différente de celle du scalp.($\sigma_4 \simeq 0$).

Du fait de ces importante discontinuités de conductivité, la contribution en sortie des ondes transmises intermédiaires peut être négligée. On ne considère donc, en sortie, que la contribution des ondes $D_{nm}$ ayant subi n réflexions entre les deux couches 31, 32 du côté interne et m réflexions entre les deux couches 32, 33 du côté externe.

La position $M_{nm}$ sur le segment $OM_o$ de l'image (source virtuelle) dont est issue l'onde $D_{nm}$ est donnée par

$$\frac{1}{OM_{nm}} = \frac{1}{b} + 2n\left(\frac{1}{a_1} - \frac{1}{a_2}\right) + 2m\left(\frac{1}{a_2} - \frac{1}{a_3}\right) .$$

Quant à l'amplitude $C^t_{nm}$ de l'onde $D_{nm}$, elle est donnée par

$$C^t_{nm} = \left(\frac{OM_{nm}}{b}\right)^2 C'^t_0 (C_0)^n (C'_0)^m , \text{ avec}$$

$$C'^t_0 = \frac{8\,\sigma_1\,\sigma_2\,\sigma_3}{(\sigma_1 + \sigma_2)(\sigma_2 + \sigma_3)(\sigma_3 + \sigma_4)} ,$$

$$C'_0 = \frac{(\sigma_3 - \sigma_4)(\sigma_3 - \sigma_2)}{(\sigma_3 + \sigma_4)(\sigma_3 + \sigma_2)} ,$$

$$C_0 = \frac{(\sigma_2 - \sigma_3)(\sigma_2 - \sigma_1)}{(\sigma_2 + \sigma_3)(\sigma_2 + \sigma_1)} .$$

Le potentiel résultant à la surface de la sphère extérieure s'exprime donc par :

$$\Psi_R(\beta, b) = C'^t_0 \sum_{n=0}^{\infty} \sum_{m=0}^{\infty} (C_0)^n (C'_0)^m \left(\frac{OM_{nm}}{b}\right)^2 \Psi_0(\beta, OM_{nm})$$

$\beta$ étant l'angle formé par le rayon passant par le point considéré à la surface de la sphère par rapport au rayon $OM_0$ passant par la source, et $\Psi_0(\beta, OM_{nm})$ étant le potentiel en surface d'une sphère dû à une charge unité placée en $M_{nm}$ à la distance $OM_{nm}$ du centre de la sphère pour un milieu homogène.

$\Psi_0(\beta, OM_{nm})$ s'exprime par :

$$\Psi_0(\beta, OM_{nm}) = \Psi_0(\beta, \eta) = \frac{1}{r}$$

$$\text{où} \quad r^2 = 1 + \eta^2 - 2\eta\cos\beta \; ; \; \eta = \frac{OM_{nm}}{a_3} \; ; \text{ et}$$

$a_3$ = rayon de la sphère extérieure.

La double somme infinie donnant le potentiel $\Psi_R(\beta, b)$ peut être simplifiée par approximation par une intégrale continue de la forme

$$\Psi_R(\beta, b) = C'^t_0 \frac{cc'}{a_3^2} \int_0^1 \frac{1-t}{t} \exp\left[-\alpha\frac{(1-t)}{t}\right] \Psi_0(\beta, bt)\, dt$$

avec

$$\frac{1}{c} = 2\left(\frac{1}{a_1} - \frac{1}{a_2}\right); \; \frac{1}{c'} = 2\left(\frac{1}{a_2} - \frac{1}{a_3}\right); \; -\alpha = \frac{1}{2}\left(\frac{c}{b}\log C_0 + \frac{c'}{b}\log C'_0\right)$$

L'intégrale ci-dessus peut être calculée par la méthode des trapèzes en discrétisant la variable t, ce qui revient à placer des charges régulièrement espacées entre le centre O de la sphère et la position $M_o$ de la charge réelle avec des poids donnés par la fonction de pondération :

$$\frac{1-t}{t} \exp\left[-\alpha \frac{(1-t)}{t}\right].$$

Le modèle décrit ci-avant pour une charge ponctuelle est facilement étendu à une source dipolaire qui offre une meilleure représentation d'une source d'activités cérébrales. Le passage à la source dipolaire est obtenu numériquement en sommant les potentiels créés par deux charges $-\frac{1}{e}$ et $\frac{1}{e}$ (où 1 caractérise le moment dipolaire et e leur distance) placées à distance (b - e) et b du centre des sphères avec e très petit devant b, et en remarquant que, dans le cas d'une sphère conductrice le potentiel à la surface de celle-ci peut s'interpréter comme la somme du potentiel de deux dipôles dans l'espace libre de même conductivité soit, finalement, à partir de la charge en $M_{nm}$ :

$[\Psi_o(\beta,\eta)$ , $-\Psi_o(\beta,\eta-e)]$ et $[{}^1/_2\Psi_o(\beta, \eta/2 )$, $-{}^1/_2\Psi_o(\beta, \frac{\eta-e}{2} )]$.

A titre d'exemple, en calculant par la méthode des trapèzes l'intégrale définie plus haut donnant une approximation du potentiel résultant $\Psi_R (\beta, b)$, en utilisant cinq trapèzes (charges virtuelles placées à 3/16, 1/8, 1/4, 1/2 et 3/4 de la distance $OM_o$ à partir de O, on obtient pour le potentiel créé par un dipôle réel :

$$\psi(\beta,\eta) = \left[7 \exp\left(-\frac{7\alpha_o}{\eta}\right) + 3 \exp\left(-\frac{6\alpha_o}{\eta}\right)\right] \psi_o\left(\beta,\frac{1}{8}\eta\right) +$$

$$\left[3 \exp\left(\frac{-3\alpha_o}{\eta}\right) + \exp\left(\frac{-2\alpha_o}{\eta}\right)\right] \psi_o\left(\beta,\frac{1}{4}\eta\right) + \left[\frac{13}{3}\exp\left(-\frac{13}{3}\frac{\alpha_o}{\eta}\right) + \frac{5}{3}\exp\left(-\frac{10\alpha_o}{3\eta}\right)\right] \psi_o\left(\beta,\frac{3}{16}\eta\right)$$

$$+ \left[\exp\left(-\frac{\alpha_o}{\eta}\right)\right] \psi_o\left(\beta,\frac{1}{2}\eta\right) + \left[\frac{1}{3}\exp\left(-\frac{\alpha_o}{3\eta}\right)\right] \psi_o\left(\beta,\frac{3}{4}\eta\right), \text{ avec } \alpha_o = 0,36.$$

Le modèle de propagation qui vient d'être décrit permet donc de définir un modèle de vecteur source $\overset{\circ}{S}(\varphi)$ dont les composantes représentent les valeurs des potentiels recueillis (à une constante près) sur les K électrodes en fonction des coordonnées de la source, coordonnées exprimées par un vecteur de position $\varphi$ relatif à la source. En d'autres termes, le vecteur source $\overset{\circ}{S}(\varphi)$ caractérise la fonction de transfert dans le domaine fréquentiel entre la position de la source et les signaux EEG recueillis sur les électrodes.

Le modèle de vecteur source $\overset{\circ}{S}(\varphi)$ correspondant au modèle de propagation sélectionné est enregistré en mémoire ROM du dispositif de traitement pour être utilisé aux fins de localisation des sources identifiées dans la matrice interspectrale débarrassée du bruit estimée $\hat{\hat{\Gamma}}_y$.

Localisation des sources

De préférence, la localisation des sources est effectuée par mise en oeuvre de la méthode du projecteur orthogonal, ou encore la méthode du goniomètre généralisée (phase 103).

Ayant constaté que pour le modèle dipolaire, le vecteur source est indépendant de la fréquence, on localisera la source, non pour chaque plan fréquentiel de la matrice $\hat{\hat{\Gamma}}_y$, mais pour une matrice $\hat{\hat{\Gamma}}_y^m$ obtenue par moyennage sur les différents plans frequentiels.

La matrice $\hat{\hat{\Gamma}}_y^m$ possède $(K - r_o)$ valeurs propres qui sont voisines de zéro (si l'estimation était parfaite, ces $(K - r_o)$ valeurs propres seraient nulles). A ces $(K - r_o)$ valeurs propres correspondent des vecteurs propres $\{\hat{\hat{v}}_i\}$ qui sont orthogonaux aux vecteurs sources $\{\hat{\hat{s}}_i\}$ qui forment le sous-espace source de dimension $r_o$.

On désigne par $\rho(\varphi)$ la fonction obtenue en projetant le modèle du vecteur source $\overset{\circ}{S}(\varphi)$ sur les valeurs propres $\{\hat{\hat{v}}_i\}$, avec normalisation par $\overset{\circ}{S}(\varphi)$ :

$$\rho\,(\varphi) = \sum_{j=r_0+1}^{K} \left| \hat{\hat{v}}_j^{\,+}\; \hat{\hat{s}}\,(\varphi) \right|^2 \Big/ \left| \hat{\hat{s}}\,(\varphi) \right|^2$$

$\hat{\hat{v}}_j^{+}$ étant le conjugué transposé de $\hat{\hat{v}}_j$.

A chaque fois que le vecteur de position $\varphi$ sera égal au vecteur de position $\varphi_i$ d'une source, la fonction $\rho(\varphi)$ doit être nulle à cause des fluctuations statistiques. C'est donc la recherche des minima de cette fonction (ou les maxima de son inverse) qui donnera les vecteurs de position recherchés $\varphi_i$ des sources. On notera que, dans l'expression ci-dessus de $\rho(\varphi)$, la normalisation par $|\hat{s}\,(\varphi)|$ est introduite pour tenir compte du fait que la norme des vecteurs souces n'est pas obligatoirement constante.

La recherche de minima de $\rho(\varphi)$ est effectuée en balayant l'ensemble des valeurs du vecteur de position .

En variante, la localisation des sources pourra être effectuée en utilisant le sous-espace source. Une méthode peut consister alors à minimiser la distance entre

$$\hat{\hat{\Gamma}}_y = \sum_{i=1}^{r_0} \hat{\hat{\gamma}}_{u_i} \hat{\hat{o}}\,(\varphi_i)\, \hat{\hat{o}}^{+}(\varphi_i) \quad \text{et} \quad \hat{\hat{\Gamma}}_y = \sum_{i=1}^{r_0} \hat{\hat{\lambda}}_i\, \hat{\hat{v}}_i\, \hat{\hat{v}}_i^{+},$$

$\hat{\hat{\lambda}}_i$ et $\hat{\hat{v}}_i$ étant les valeurs propres et vecteurs propres du sous-espace source, et

$$\hat{\hat{\gamma}}_{u_i}$$

est la densité spectrale de puissance de la source i.

Toutefois, par rapport à la méthode du projecteur orthogonal, cette méthode présente le désavantage de faire porter la recherche des paramètres de position sur tous les paramètres à la fois.

On notera que la méthode de localisation par projecteur orthogonal décrite ci-avant en liaison avec le modèle de propagation envisagé impose une disposition des électrodes dans un même plan et suppose une orientation radiale du dipôle représentant la source. Le choix du modèle dipolaire radial est ici justifié en raison de l'orientation radiale des cellules pyramidales au niveau du cortex.

La disposition des électrodes dans un plan permet une localisation uniquement dans ce plan.

Une localisation en trois dimensions est possible en effectuant des localisations dans plusieurs plans parallèles en liaison avec une cartographie donnant dans chaque plan une idée sur les niveaux de puissance d'émission.

La localisation en trois dimensions peut être directement obtenue en élaborant un modèle de propagation plus complexe faisant intervenir un paramètre de position supplémentaire et une orientation quelconque du (ou des) dipôles.

Exemple

Les figures 4A à 4E se rapportent à un exemple de mise en oeuvre du dispositif selon l'invention pour la localisation d'un foyer épileptique.

La figure 4A montre les emplacements des 16 électrodes utilisées.

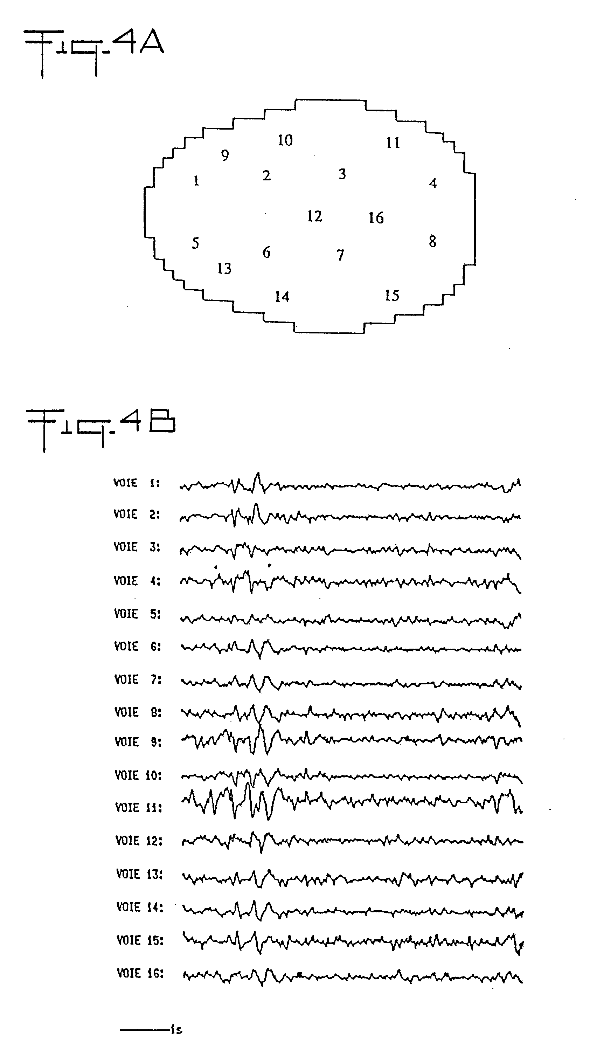

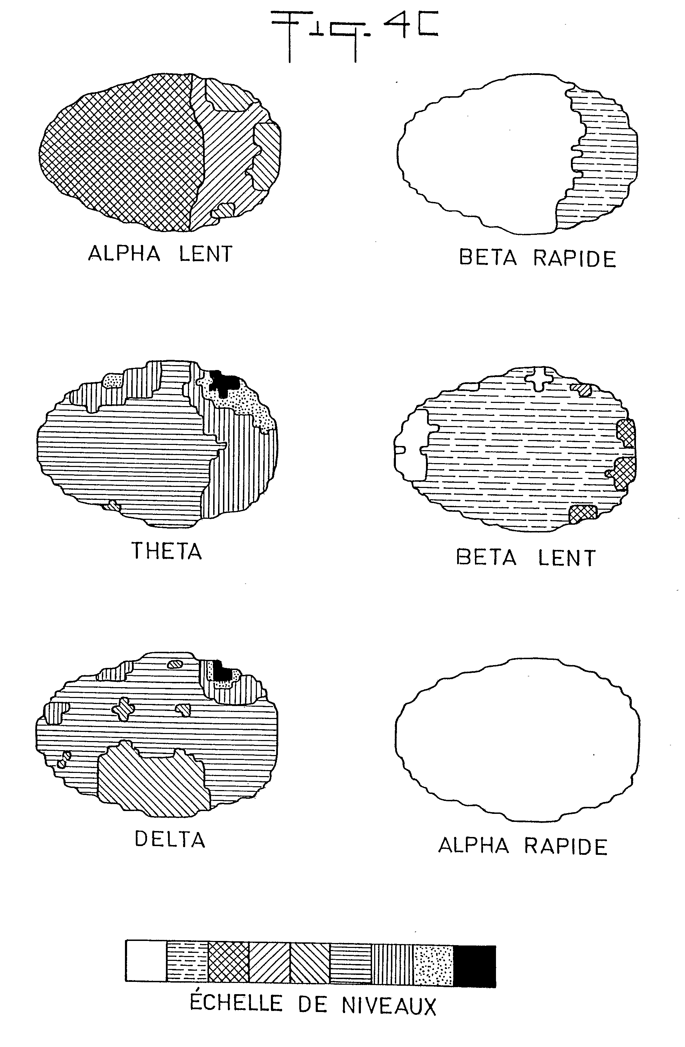

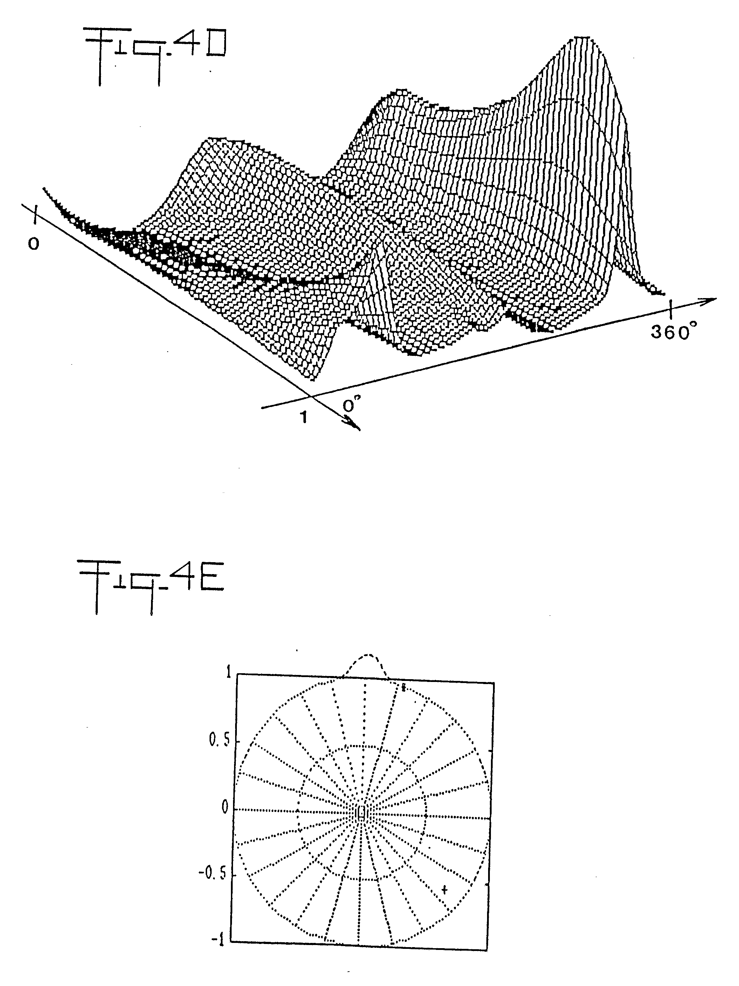

La figure 4B montre les signaux analogiques EEG recueillis sur les 16 électrodes.

Une matrice interspectrale $\hat{\hat{\Gamma}}_x$ a été estimée à partir des signaux EEG recueillis par les électrodes coplanaires No 4, 10, 9, 13, 14 et 8.

La diagonalisation de la matrice de cohérence $\hat{\hat{c}}_x$ associée à la matrice interspectrale estimée $\hat{\hat{\Gamma}}_x$, dans la bande delta (0,1 à 4 Hz) fait apparaître six valeurs propres non nulles (autant que de capteurs) dont deux paraissent prépondérantes.

Une matrice $\hat{\hat{\Gamma}}_y$ débarrassée du bruit a été estimée à partir de $\hat{\hat{\Gamma}}_x$ comme décrit plus haut. De l'estimation de $\hat{\hat{\Gamma}}_y$, il ressort que la matrice a un rang égal à deux. La diagonalisation de la matrice de cohérence $\hat{\hat{c}}_y$ associée à $\hat{\hat{\Gamma}}_y$ fait apparaître deux valeurs propres distinctes auxquelles peuvent être associées deux sources décorrélées.

La localisation des deux sources a été effectuée par recherche des maxima de la fonction $1/\rho(\varphi)$, ce qui est équivalent à la recherche des minima de $\rho(\varphi)$, en utilisant la méthode du projecteur orthogonal avec deux vecteurs source élaborés à partir du modèle de propagation à trois discontinuités de conductivité, comme décrit plus haut.

La figure 4C montre la cartographie réalisée à partir de la matrice interspectrale estimée $\hat{\Gamma}_x$, dans les bandes alpha lent (7,5 à 10 Hz), bêta rapide (18 à 40 Hz), thêta (4 à 7,5 Hz), bêta lent (12,5 à 18 Hz), delta (0,1 à 4 Hz) et alpha rapide (10 à 12,5 Hz).

Les cartographies dans la bande thêta et delta indiquent la présence d'un foyer épileptique dans la région temporale droite.

La figure 4D montre la variation de la fonction $1/\rho(\varphi)$ dans le plan défini par les électrodes sélectionnées.

Deux maxima de la fonction $1/\rho(\varphi)$ apparaissent sur la figure 4D, les positions de ces maxima étant données sur la figure 4E par une croix et un astérisque. Sur la figure 4E, l'axe horizontal passant par le centre O correspond à celui des électrodes No 14 et 10. La croix correspond à la localisation du foyer épileptique, tandis que l'astérisque reflète vraisemblablement une "source" relative à des mouvements oculaires.

## Revendications

1. Dispositif de stéréolocalisation spatiale de sources d'activités cérébrales, caractérisé en ce qu'il comporte :

(a) un ensemble d'électrodes (10) destinées à être disposées en des emplacements prédéterminés du scalp pour recueillir des signaux EEG,

(b) des moyens convertisseurs (14) pour convertir les signaux EEG recueillis sous forme numérique, et

(c) une unité de traitement comprenant :

- des moyens d'estimation optimale d'une matrice interspectrale des signaux EEG recueillis,

- des moyens de mémorisation dans lesquels sont enregistrées des informations relatives à un modèle de propagation et permettant de caractériser au moins un vecteur source définissant des relations, fonctions de paramètres de position de la ou chaque source, dans le domaine fréquentiel, entre un signal émis par une source dipolaire ou chaque source et les signaux disponibles sur le scalp, le modèle de propagation étant un modèle à trois discontinuités comprenant le cerveau, le crâne et le scalp, et

- des moyens de traitement de la matrice interspectrale estimée pour, d'une part, identifier les sources d'activités cérébrales contribuant aux signaux EEG recueillis et déterminer leur nombre et, d'autre part, estimer les coordonnées de chaque source identifiée.

2. Dispositif de stéréolocalisation selon la revendication 1, caractérisé en ce que les moyens de traitement de la matrice interspectrale comprennent des moyens d'estimation, sur la matrice interspectrale débarrassée du bruit, des vecteurs propres correspondant aux valeurs propres de la matrice non liées aux sources identifiées, et des moyens de détermination des coordonnées de position de source pour lesquelles le vecteur source correspondant est orthogonal auxdits vecteurs propres.

3. Dispositif de stéréolocalisation selon l'une quelconque des revendications 1 et 2, caractérisé en ce que les moyens de d'estimation de la matrice interspectrale débarrassée du bruit, à partir de la matrice de cohérence associée à la matrice interspectrale.

TRANSFORMATEUR DE FOURIER

CALCUL PÉRIODO-GRAMME, INTER-PÉRIODOGRAMME ET PRÉMOYEN-NAGE

LISSAGE OPTIMAL

CALCUL DE LA MATRICE INTER-SPECTRALE DÉBAR-RASSÉE DU BRUIT

CALCUL DU PRO-JECTEUR ORTHO-GONAL ASSOCIÉ AUX VECTEURS SOURCES

LOCALISATION DES SOURCES

MULTIPLEXEUR

CA/N

E/S

PROCESSEUR MAÎTRE

ROM

RAM

Fig 1

## Fig. 2

```
┌──────────────────────────────────┐
│   ESTIMATION DE LA MATRICE        │
│                          ^        │────── 101
│   INTERSPECTRALE       Π̂_X        │
└──────────────────────────────────┘
                 │
                 ▼
┌──────────────────────────────────┐
│   ESTIMATION DE LA MATRICE        │
│   INTERSPECTRALE DÉBARRASSÉE       │────── 102
│                          ^        │
│   DU  BRUIT            Π̂_y        │
└──────────────────────────────────┘
                 │
                 ▼
┌──────────────────────────────────┐
│   LOCALISATION DES SOURCES PAR    │
│   LA MÉTHODE DU PROJECTEUR        │
│   ORTHOGONAL POUR UN MODÈLE       │────── 103
│   DE PROPAGATION À  3             │
│   DISCONTINUITÉS                  │
└──────────────────────────────────┘
```

## Fig. 3

Fig.4A

Fig.4B

VOIE 1:

VOIE 2:

VOIE 3:

VOIE 4:

VOIE 5:

VOIE 6:

VOIE 7:

VOIE 8:

VOIE 9:

VOIE 10:

VOIE 11:

VOIE 12:

VOIE 13:

VOIE 14:

VOIE 15:

VOIE 16:

1s

Fig. 4C

ALPHA LENT

BETA RAPIDE

THETA

BETA LENT

DELTA

ALPHA RAPIDE

ÉCHELLE DE NIVEAUX

Fig.4D

Fig.4E

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 241 757 (DUFFY)<br>* Page 1, ligne 28 – page 18, ligne 23;<br>page 21, ligne 5 – page 22, ligne 29;<br>page 39, ligne 10 – page 46, ligne 21;<br>page 54, ligne 1 – page 59, ligne 5;<br>page 67, ligne 22 – page 70, ligne 9;<br>figure 1 *<br>--- | 1 | G 06 F 15/20 |
| A | WO-A-8 303 745 (SALB)<br>* Page 2, ligne 23 – page 3, ligne 25;<br>page 4, ligne 24 – page 6, ligne 17;<br>figure 1 *<br>--- | 1 | |
| A | EP-A-0 150 125 (COHEN)<br>* Page 5, ligne 18 – page 8, ligne 13;<br>page 9, ligne 5 – page 10, ligne 29;<br>page 18, ligne 26 – page 21, ligne 20;<br>figures 1,4 *<br>--- | 1 | |
| A | EP-A-0 013 183 (JOHN)<br>* Page 4, ligne 18 – page 6, ligne 28;<br>page 7, ligne 6 – page 14, ligne 13;<br>figures 1,2 *<br>----- | 1 | DOMAINES TECHNIQUES RECHERCHES (Int. Cl.4)<br><br>G 06 F 15/20 |

Le présent rapport a été établi pour toutes les revendications

| Lieu de la recherche | Date d'achèvement de la recherche | Examinateur |
|---|---|---|
| LA HAYE | 02-02-1989 | CHUGG D.J. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P0402)